# FASCICULE DE BREVET EUROPEEN

(11) **EP 1 670 309 B1**
(45) Date de publication et mention de la délivrance du brevet: **23.06.2010**
(21) Numéro de dépôt: 04787459.9
(22) Date de dépôt: 27.09.2004
(51) Int. Cl.: A01K 67/027, C07K 14/47, G01N 33/50, C12N 15/90, C12N 5/10, C12N 15/12, C12N 15/63, C12Q 1/02

(54) **ANIMAUX TRANSGENIQUES PRESENTANT LES TROUBLES MAJEURS LIES A LA MALADIE D'ALZHEIMER**
TRANSGENE TIERE MIT SCHWEREN PATHOLOGISCHEN ZUSTÄNDEN, DIE MIT MORBUS ALZHEIMER EINHERGEHEN
TRANSGENIC ANIMALS WITH SERIOUS DISORDERS RELATED TO ALZHEIMER'S DISEASE

(30) Priorité: 02.10.2003 FR 0311578
(43) Date de publication de la demande: 21.06.2006
(73) Titulaire: Aventis Pharma S.A., 92160 Antony (FR)
(72) Inventeur: CASAS LOUZAO, Caty, E-08030 Barcelone (ES); BENOIT, Patrick, F-75014 Paris (FR); PRADIER, Laurent, F-91370 Verrieres (FR); TREMP, Gunter, F-91120 Palaiseau (FR); ITIER, Jean-Michel, F-91600 Savigny sur Orge (FR); BLANCHARD-BREGEON, Véronique, F-75012 Paris (FR)
(74) Mandataire: Bouvet, Philippe
(86) Numéro de dépôt international: PCT/FR2004/002436
(87) Numéro de publication internationale: WO 2005/032244

(56) Documents cités:
- WO-A-01/20977
- WO-A-02/08407
- FR-A- 2 798 556
- HUANG X G ET AL: "Behavioral and neurochemical characterization of transgenic mice carrying the human presenilin-1 gene with or without the leucine-to-proline mutation at codon 235." EXPERIMENTAL NEUROLOGY, vol. 183, no. 2, octobre 2003 (2003-10), pages 673-681, XP002290682 ISSN: 0014-4886
- DATABASE BIOSIS [Online] BIOSCIENCES INFORMATION SERVICE, PHILADELPHIA, PA, US; 2002, KADISH I ET AL: "ALZHEIMER GENES INCREASE AXONAL SPROUTING IN APP/PS1 MUTANT MICE." XP002290686 Database accession no. PREV200300293435 & SOCIETY FOR NEUROSCIENCE ABSTRACT VIEWER AND ITINERARY PLANNER, vol. 2002, 2002, page Abstract No. 295.7 URL, 32ND ANNUAL MEETING OF THE SOCIETY FOR NEUROSCIENCE; ORLANDO, FLORIDA, USA; NOVEMBER 02-07, 2002
- DATABASE BIOSIS [Online] BIOSCIENCES INFORMATION SERVICE, PHILADELPHIA, PA, US; 2001, STEINER B R ET AL: "Antioxidant enzyme activities in brains of mice transgenic for wildtype and mutant human presenilin-1 and/or mutant human amyloid precursor protein" XP002290687 Database accession no. PREV200100532603 & SOCIETY FOR NEUROSCIENCE ABSTRACTS, vol. 27, no. 1, 2001, page 1136, 31ST ANNUAL MEETING OF THE SOCIETY FOR NEUROSCIENCE; SAN DIEGO, CALIFORNIA, USA; NOVEMBER 10-15, 2001 ISSN: 0190-5295
- KWOK J B ET AL: "Two novel (M233T and R278T) presenilin-1 mutations in early-onset Alzheimer's disease pedigrees and preliminary evidence for association of presenilin-1 mutations with a novel phenotype." NEUROREPORT. 14 APR 1997, vol. 8, no. 6, 14 avril 1997 (1997-04-14), pages 1537-1542, XP009034735 ISSN: 0959-4965 cité dans la demande
- CAMPION D ET AL: "A novel presenilin 1 mutation resulting in familial Alzheimer's disease with an onset age of 29 years" NEUROREPORT 1996 UNITED KINGDOM, vol. 7, no. 10, 1996, pages 1582-1584, XP009034736 ISSN: 0959-4965 cité dans la demande
- BLANCHARD VÉRONIQUE ET AL: "Time sequence of maturation of dystrophic neurites associated with Abeta deposits in APP/PS1 transgenic mice." EXPERIMENTAL NEUROLOGY. NOV 2003, vol. 184, no. 1, novembre 2003 (2003-11), pages 247-263, XP002290685 ISSN: 0014-4886

### SEQUENCE LISTING

<110> AVENTIS PHARMA S.A.
<120> ANIMAUX TRANSGENIQUES EXPRIMANT DES MUTANTS DE PS1 ET APP PRESENTANT LES TROUBLES MAJEURS LIES A LA MALADIE D'ALZHEIMER
<130> PRJ03034
<160> 9
<170> PatentIn version 3.1
<210> 1
   <211> 27
   <212> DNA
   <213> Mus musculus
<400> 1
   atcagtgccc tcacggcacc ggtattt 27
<210> 2
   <211> 9
   <212> PRT
   <213> Mus musculus
<400> 2
   Ile Ser Ala Leu Thr Ala Pro Val Phe
   1               5
<210> 3
   <211> 467
   <212> PRT
   <213> Mus musculus
<400> 3
   Met Thr Glu Ile Pro Ala Pro Leu Ser Tyr Phe Gln Asn Ala Gln Met
   1               5                   10                  15
   Ser Glu Asp Ser His Ser Ser Ser Ala Ile Arg Ser Gln Asn Asp Ser
               20                  25                  30
   Gln Glu Arg Gln Gln Gln His Asp Arg Gln Arg Leu Asp Asn Pro Glu
           35                  40                  45
   Pro Ile Ser Asn Gly Arg Pro Gln Ser Asn Ser Arg Gln Val Val Glu
       50                  55                  60
   Gln Asp Glu Glu Glu Asp Glu Glu Leu Thr Leu Lys Tyr Gly Ala Lys
   65                  70                  75                  80
   His Val Ile Met Leu Phe Val Pro Val Thr Leu Cys Met Val Val Val
                   85                  90                  95
   Val Ala Thr Ile Lys Ser Val Ser Phe Tyr Thr Arg Lys Asp Gly Gln
               100                 105                 110
   Leu Ile Tyr Thr Pro Phe Thr Glu Asp Thr Glu Thr Val Gly Gln Arg
           115                 120                 125
   Ala Leu His Ser Ile Leu Asn Ala Ala Ile Met Ile Ser Val Ile Val
       130                 135                 140
   Ile Met Thr Ile Leu Leu Val Val Leu Tyr Lys Tyr Arg Cys Tyr Lys
   145                 150                 155                 160
   Val Ile His Ala Trp Leu Ile Ile Ser Ser Leu Leu Leu Leu Phe Phe
                   165                 170                 175
   Phe Ser Phe Ile Tyr Leu Gly Glu Val Phe Lys Thr Tyr Asn Val Ala
               180                 185                 190
   Val Asp Tyr Val Thr Val Ala Leu Leu Ile Trp Asn Phe Gly Val Val
           195                 200                 205
   Gly Met Ile Ala Ile His Trp Lys Gly Pro Leu Arg Leu Gln Gln Ala
       210                 215                 220
   Tyr Leu Ile Met Ile Ser Ala Leu Thr Ala Pro Val Phe Ile Lys Tyr
   225                 230                 235                 240
   Leu Pro Glu Trp Thr Ala Trp Leu Ile Leu Ala Val Ile Ser Val Tyr
                   245                 250                 255
   Asp Leu Val Ala Val Leu Cys Pro Lys Gly Pro Leu Arg Met Leu Val
               260                 265                 270
   Glu Thr Ala Gln Glu Arg Asn Glu Thr Leu Phe Pro Ala Leu Ile Tyr
                   275                 280                 285
   Ser Ser Thr Met Val Trp Leu Val Asn Met Ala Glu Gly Asp Pro Glu
       290                 295                 300
   Ala Gln Arg Arg Val Pro Lys Asn Pro Lys Tyr Asn Thr Gln Arg Ala
   305                 310                 315                 320
   Glu Arg Glu Thr Gln Asp Ser Gly Ser Gly Asn Asp Asp Gly Gly Phe
                   325                 330                 335
   Ser Glu Glu Trp Glu Ala Gln Arg Asp Ser His Leu Gly Pro His Arg
               340                 345                 350
   Ser Thr Pro Glu Ser Arg Ala Ala Val Gln Glu Leu Ser Gly Ser Ile
           355                 360                 365
   Leu Thr Ser Glu Asp Pro Glu Glu Arg Gly Val Lys Leu Gly Leu Gly
       370                 375                 380
   Asp Phe Ile Phe Tyr Ser Val Leu Val Gly Lys Ala Ser Ala Thr Ala
   385                 390                 395                 400
   Ser Gly Asp Trp Asn Thr Thr Ile Ala Cys Phe Val Ala Ile Leu Ile
                   405                 410                 415
   Gly Leu Cys Leu Thr Leu Leu Leu Leu Ala Ile Phe Lys Lys Ala Leu
               420                 425                 430
   Pro Ala Leu Pro Ile Ser Ile Thr Phe Gly Leu Val Phe Tyr Phe Ala
           435                 440                 445
   Thr Asp Tyr Leu Val Gln Pro Phe Met Asp Gln Leu Ala Phe His Gln
       450                 455                 460
   Phe Tyr Ile
   465
<210> 4
   <211> 27
   <212> DNA
   <213> Mus musculus
<400> 4
   atcagtgccc tcatggccct ggtattt 27
<210> 5
   <211> 9
   <212> PRT
   <213> Mus musculus
<400> 5
   Ile Ser Ala Leu Met Ala Leu Val Phe
   1               5
<210> 6
   <211> 2265
   <212> DNA
   <213> Homo sapiens
<400> 6
   cccgggtcca ccatgctgcc cggtttggca ctgctcctgc tggccgcctg gacggctcgg    60
   gcgctggagg tacccactga tggtaatgct ggcctgctgg ctgaacccca gattgccatg   120
   ttctgtggca gactgaacat gcacatgaat gtccagaatg ggaagtggga ttcagatcca   180
   tcagggacca aaacctgcat tgataccaag gaaggcatcc tgcagtattg ccaagaagtc   240
   taccctgaac tgcagatcac caatgtggta gaagccaacc aaccagtgac catccagaac   300
   tggtgcaagc ggggccgcaa gcagtgcaag acccatcccc actttgtgat tccctaccgc   360
   tgcttagttg gtgagtttgt aagtgatgcc cttctcgttc ctgacaagtg caaattctta   420
   caccaggaga ggatggatgt ttgcgaaact catcttcact ggcacaccgt cgccaaagag   480
   acatgcagtg agaagagtac caacttgcat gactacggca tgttgctgcc ctgcggaatt   540
   gacaagttcc gaggggtaga gtttgtgtgt tgcccactgg ctgaagaaag tgacaatgtg   600
   gattctgctg atgcggagga ggatgactcg gatgtctggt ggggcggagc agacacagac   660
   tatgcagatg ggagtgaaga caaagtagta gaagtagcag aggaggaaga agtggctgag   720
   gtggaagaag aagaagccga tgatgacgag gacgatgagg atggtgatga ggtagaggaa   780
   gaggctgagg aaccctacga agaagccaca gagagaacca ccagcattgc caccaccacc   840
   accaccacca cagagtctgt ggaagaggtg gttcgagagg tgtgctctga acaagccgag   900
   acggggccgt gccgagcaat gatctcccgc tggtactttg atgtgactga agggaagtgt   960
   gccccattct tttacggcgg atgtggcggc aaccggaaca actttgacac agaagagtac  1020
   tgcatggccg tgtgtggcag cgccattcct acaacagcag ccagtacccc tgatgccgtt  1080
   gacaagtatc tcgagacacc tggggatgag aatgaacatg cccatttcca gaaagccaaa  1140
   gagaggcttg aggccaagca ccgagagaga atgtcccagg tcatgagaga atgggaagag  1200
   gcagaacgtc aagcaaagaa cttgcctaaa gctgataaga aggcagttat ccagcatttc  1260
   caggagaaag tggaatcttt ggaacaggaa gcagccaacg agagacagca gctggtggag  1320
   acacacatgg ccagagtgga agccatgctc aatgaccgcc gccgcctggc cctggagaac 1380
   tacatcaccg ctctgcaggc tgttcctcct cggcctcgtc acgtgttcaa tatgctaaag 1440
   aagtatgtcc gcgcagaaca gaaggacaga cagcacaccc taaagcattt cgagcatgtg 1500
   cgcatggtgg atcccaagaa agccgctcag atccggtccc aggttatgac acacctccgt 1560
   gtgatttatg agcgcatgaa tcagtctctc tccctgctct acaacgtgcc tgcagtggcc 1620
   gaggagattc aggatgaagt tgatgagctg cttcagaaag agcaaaacta ttcagatgac 1680
   gtcttggcca acatgattag tgaaccaagg atcagttacg gaaacgatgc tctcatgcca 1740
   tctttgaccg aaacgaaaac caccgtggag ctccttcccg tgaatggaga gttcagcctg 1800
   gacgatctcc agccgtggca ttcttttggg gctgactctg tgccagccaa cacagaaaac 1860
   gaagttgagc ctgttgatgc ccgccctgct gccgaccgag gactgaccac tcgaccaggt 1920
   tctgggttga caaatatcaa gacggaggag atctctgaag tgaatctgga tgcagaattc 1980
   cgacatgact caggatatga agttcatcat caaaaattgg tgttctttgc agaagatgtg 2040
   ggttcaaaca aaggtgcaat cattggactc atggtgggcg gtgttgtcat agcgacagtg 2100
   atcatcatca ccttggtgat gctgaagaag aaacagtaca catccattca tcatggtgtg 2160
   gtggaggttg acgccgctgt caccccagag gagcgccacc tgtccaagat gcagcagaac 2220
   ggctacgaaa atccaaccta caagttcttt gagcagatgc agaac 2265
<210> 7
   <211> 751
   <212> PRT
   <213> Homo sapiens
<400> 7
   Met Leu Pro Gly Leu Ala Leu Leu Leu Leu Ala Ala Trp Thr Ala Arg
   1 5 10 15
   Ala Leu Glu Val Pro Thr Asp Gly Asn Ala Gly Leu Leu Ala Glu Pro
   20 25 30
   Gln Ile Ala Met Phe Cys Gly Arg Leu Asn Met His Met Asn Val Gln
   35 40 45
   Asn Gly Lys Trp Asp Ser Asp Pro Ser Gly Thr Lys Thr Cys Ile Asp
   50 55 60
   Thr Lys Glu Gly Ile Leu Gln Tyr Cys Gln Glu Val Tyr Pro Glu Leu
   65 70 75 80
   Gln Ile Thr Asn Val Val Glu Ala Asn Gln Pro Val Thr Ile Gln Asn
   85 90 95
   Trp Cys Lys Arg Gly Arg Lys Gln Cys Lys Thr His Pro His Phe Val
               100                 105                 110
   Ile Pro Tyr Arg Cys Leu Val Gly Glu Phe Val Ser Asp Ala Leu Leu
           115                 120                 125
   Val Pro Asp Lys Cys Lys Phe Leu His Gln Glu Arg Met Asp Val Cys
       130                 135                 140
   Glu Thr His Leu His Trp His Thr Val Ala Lys Glu Thr Cys Ser Glu
   145                 150                 155                 160
   Lys Ser Thr Asn Leu His Asp Tyr Gly Met Leu Leu Pro Cys Gly Ile
                   165                 170                 175
   Asp Lys Phe Arg Gly Val Glu Phe Val Cys Cys Pro Leu Ala Glu Glu
               180                 185                 190
   Ser Asp Asn Val Asp Ser Ala Asp Ala Glu Glu Asp Asp Ser Asp Val
           195                 200                 205
   Trp Trp Gly Gly Ala Asp Thr Asp Tyr Ala Asp Gly Ser Glu Asp Lys
       210                 215                 220
   Val Val Glu Val Ala Glu Glu Glu Glu Val Ala Glu Val Glu Glu Glu
   225                 230                 235                 240
   Glu Ala Asp Asp Asp Glu Asp Asp Glu Asp Gly Asp Glu Val Glu Glu
                   245                 250                 255
   Glu Ala Glu Glu Pro Tyr Glu Glu Ala Thr Glu Arg Thr Thr Ser Ile
               260                 265                 270
   Ala Thr Thr Thr Thr Thr Thr Thr Glu Ser Val Glu Glu Val Val Arg
           275                 280                 285
   Glu Val Cys Ser Glu Gln Ala Glu Thr Gly Pro Cys Arg Ala Met Ile
       290                 295                 300
   Ser Arg Trp Tyr Phe Asp Val Thr Glu Gly Lys Cys Ala Pro Phe Phe
   305                 310                 315                 320
   Tyr Gly Gly Cys Gly Gly Asn Arg Asn Asn Phe Asp Thr Glu Glu Tyr
                   325                 330                 335
   Cys Met Ala Val Cys Gly Ser Ala Ile Pro Thr Thr Ala Ala Ser Thr
               340                 345                 350
   Pro Asp Ala Val Asp Lys Tyr Leu Glu Thr Pro Gly Asp Glu Asn Glu
           355                 360                 365
   His Ala His Phe Gln Lys Ala Lys Glu Arg Leu Glu Ala Lys His Arg
   370 375 380
   Glu Arg Met Ser Gln Val Met Arg Glu Trp Glu Glu Ala Glu Arg Gln
   385 390 395 400
   Ala Lys Asn Leu Pro Lys Ala Asp Lys Lys Ala Val Ile Gln His Phe
   405 410 415
   Gln Glu Lys Val Glu Ser Leu Glu Gln Glu Ala Ala Asn Glu Arg Gln
   420 425 430
   Gln Leu Val Glu Thr His Met Ala Arg Val Glu Ala Met Leu Asn Asp
   435 440 445
   Arg Arg Arg Leu Ala Leu Glu Asn Tyr Ile Thr Ala Leu Gln Ala Val
   450 455 460
   Pro Pro Arg Pro Arg His Val Phe Asn Met Leu Lys Lys Tyr Val Arg
   465 470 475 480
   Ala Glu Gln Lys Asp Arg Gln His Thr Leu Lys His Phe Glu His Val
   485 490 495
   Arg Met Val Asp Pro Lys Lys Ala Ala Gln Ile Arg Ser Gln Val Met
   500 505 510
   Thr His Leu Arg Val Ile Tyr Glu Arg Met Asn Gln Ser Leu Ser Leu
   515 520 525
   Leu Tyr Asn Val Pro Ala Val Ala Glu Glu Ile Gln Asp Glu Val Asp
   530 535 540
   Glu Leu Leu Gln Lys Glu Gln Asn Tyr Ser Asp Asp Val Leu Ala Asn
   545 550 555 560
   Met Ile Ser Glu Pro Arg Ile Ser Tyr Gly Asn Asp Ala Leu Met Pro
   565 570 575
   Ser Leu Thr Glu Thr Lys Thr Thr Val Glu Leu Leu Pro Val Asn Gly
   580 585 590
   Glu Phe Ser Leu Asp Asp Leu Gln Pro Trp His Ser Phe Gly Ala Asp
   595 600 605
   Ser Val Pro Ala Asn Thr Glu Asn Glu Val Glu Pro Val Asp Ala Arg
   610 615 620
   Pro Ala Ala Asp Arg Gly Leu Thr Thr Arg Pro Gly Ser Gly Leu Thr
   625 630 635 640
   Asn Ile Lys Thr Glu Glu Ile Ser Glu Val Asn Leu Asp Ala Glu Phe
                   645                 650                 655
   Arg His Asp Ser Gly Tyr Glu Val His His Gln Lys Leu Val Phe Phe
               660                 665                 670
   Ala Glu Asp Val Gly Ser Asn Lys Gly Ala Ile Ile Gly Leu Met Val
           675                 680                 685
   Gly Gly Val Val Ile Ala Thr Val Ile Ile Ile Thr Leu Val Met Leu
       690                 695                 700
   Lys Lys Lys Gln Tyr Thr Ser Ile His His Gly Val Val Glu Val Asp
   705                 710                 715                 720
   Ala Ala Val Thr Pro Glu Glu Arg His Leu Ser Lys Met Gln Gln Asn
                   725                 730                 735
   Gly Tyr Glu Asn Pro Thr Tyr Lys Phe Phe Glu Gln Met Gln Asn
               740                 745                 750
<210> 8
   <211> 221
   <212> DNA
   <213> Mus musculus
<400> 8
   ggaagtattt aagacctaca atgtcgccgt ggactacgtt acagtagcac tcctaatctg     60
   gaattttggt gtggtcggga tgattgccat ccactggaaa ggcccccttc gactgcagca    120
   ggcgtatctc attatgatca gtgccctcac ggcaccggta tttatcaagt acctccccga    180
   atggaccgca tggctcatct tggctgtgat ttcagtatat g                        221
<210> 9
   <211> 221
   <212> DNA
   <213> Mus musculus
<400> 9
   ggaagtattt aagacctaca atgtcgccgt ggactacgtt acagtagcac tcctaatctg     60
   gaattttggt gtggtcggga tgattgccat ccactggaaa ggcccccttc gactgcagca    120
   ggcgtatctc attatgatca gtgccctcat ggccctggta tttatcaagt acctccccga    180
   atggaccgca tggctcatct tggctgtgat ttcagtatat g                        221

## Description

La présente demande est relative à des animaux transgèniques modèles de la maladie d'Alzheimer (AD). Elle a en outre pour objet l'utilisation de ces animaux.

La maladie d'Alzheimer est une maladie neurodégénérative progressive qui affecte une large proportion de la population âgée. Cette maladie est caractérisée sur le plan clinique par une perte de la mémoire et un déclin des fonctions cognitives, et sur le plan neuropathologique par une perte neuronale prononcée et la présence dans le cerveau de dépôts neurofibrillaires intracellulaires et de dépôts extracellulaires du peptide β-amyloïde (Aβ) formant des plaques amyloïdes.

Les plaques amyloïdes sont majoritairement composées des peptides Aβ à 40 ou 42 résidus qui sont générés lors du processus protéolytique du précurseur du peptide Aβ (APP). Les dépôts extracellulaires de Aβ sont très spécifiques des désordres associés à la maladie d'Alzheimer. Ils représentent la caractéristique précoce et invariable de toutes les formes de la maladie d'Alzheimer, incluant les formes familiales (FAD). Les formes familiales apparaissent de manière relativement précoce (entre 30 et 60 ans) et sont dues à des mutations dans le gène de l'APP dans 5 % des cas de FAD avec huit mutations faux-sens simples ou doubles identifiées, dans le gène de la préséniline 1 (PS1) dans 50 à 70% des cas de FAD avec plus de 100 mutations différentes identifiées jusqu'à présent, et dans le gène de la préséniline 2 dans des cas plus rares de FAD avec deux mutations faux sens décrites. On a montré que des mutations dans ces trois gènes induisent des changements dans la protéolyse de l'APP, qui conduisent à une surproduction de l'Aβ, spécialement de la forme longue Aβ42, et à l'apparition précoce de la pathologie et des symptômes similaires à ceux des formes sporadiques de la maladie d'Alzheimer.

Des modèles animaux destinés à représenter certaines caractéristiques de la pathologie de la maladie d'Alzheimer ont déjà été décrits dans la littérature.

Il s'agit d'une part de souris transgèniques portant des mutations dans le gène APP. Elles développent des pathologies similaires à la maladie d'Alzheimer à partir d'un an. Ainsi la souris PDAPP, surexprimant l'APP humaine portant la mutation V717F, développe avec l'âge des dépôts de Aβ dans le cerveau mais ne montre pas de perte neuronale au delà de l'emplacement des plaques elles-mêmes (Irizarry et al., 1997, J. Neurosc. 17(18) : 7053-7059). Nous appellerons ce phénomène « effet plaque ».

De même, la souris Tg(HuAPP695. K670N-M671L)2576, exprimant l'isoforme humaine APP K670N-M67IL (APPSw pour mutation type Swedish), présente des dépôts types amyloïdes mais ne montre pas de perte neuronale (Irizarry et al., 1997, J. Neuropathol. Exp. Neurol 56 : 695-973).

Dans une étude de Calhoun et al. (1998, Nature 395 : 755-756), une perte neuronale a été montrée dans certaines régions cérébrales à proximité des plaques amyloïdes, chez des souris transgèniques APP23 de 14-18 mois d'âge exprimant une isoforme mutée de APP humaine. Cette observation est controversée car la perte est faible et intervient chez des animaux relativement âgés et surtout à proximité des plaques, ce qui pourrait correspondre à l' « effet plaque » observé précédemment. De plus, elle n'est que peu ou pas mentionnée dans un commentaire récent qui souligne que les modèles animaux actuels ne présentent pas une entière similitude avec toutes les caractéristiques connues des pathologies de la maladie d'Alzheimer, entre autre la perte neuronale (Trojanowski, 2002, Am. J. Pathol.160: 409-411).

D'autre part, on connaît des souris transgèniques portant des mutations dans le gène PS1. Celles ci ne semblent pas développer de pathologie type maladie d'Alzheimer mais présentent une quantité élevée de peptide Aβ42 (augmentation d'un facteur 2 par rapport à aux PS 1 sauvages) qui est reconnu comme hautement pathogénique.

De plus, dans les modèles animaux transgèniques décrits portant des mutations FAD P264L ou M146L dans le gène PS1 de souris (« knock-in »), la protéine PS1 mutée n'est pas exprimée de façon stable (Siman et al., 2000, J. Neurosci., 20 : 8717-8726 ; Flood et al., 2002, Neurobiol. Aging 23 : 335-348 ; Rozmahel et al., 2002, 23 :187-194). Ces souris présentent aussi une quantité élevée de peptide Aβ42.

La demande WO 02/0008407 décrit de telles souris transgèniques dont le gène codant la préséniline 1 a été muté par introduction d'une mutation P264L.

Huang et al. (Exp.Neurol., vol. 183, No2, Oct 2003, pp 673-681) décrit des souris transgéniques, apparemment hétérozygotes exprimant la protéine PS 1 humaine avec la mutation L235P.

Un lien possible entre les mutations respectives M233T et L235P de PS1 1 et des formes précoces de la maladie d'Alzheimer est évoqué par la littérature (Kwok et al., Neuroreport 14 avril 1997, vol 8, No 6, pp1537-1542, et Campion et al., Neuroreport 1996 UK, vol 7, No 10, 1996, pp1582-1584).

En raison du rôle de la protéine PS1 dans la formation des formes Aβ42, des souris doubles transgèniques portant des mutations dans les gènes APP et PS1 ont aussi été fabriquées. Comme les simples transgèniques décrites plus haut, ces souris présentent des dépôts d'Aβ mais ne présentent pas de perte neuronale (Takeuchi et al., 2000, Am. J. Pathol. 157 : 331-339).

Ainsi les modèles animaux de la maladie d'Alzheimer existant ne sont pas satisfaisants car ils échouent à reproduire une perte neuronale qui est pourtant une caractéristique majeure des maladies neurodégénératives, dont la maladie d'Alzheimer.

La demanderesse s'est donc attachée à fabriquer des animaux présentant des caractéristiques majeures de la maladie d'Alzheimer, dont la perte neuronale.

Elle a montré qu'il était possible d'obtenir de tels animaux en introduisant des mutations spécifiques dans le gène codant la protéine PS1 chez la souris et en les croisant avec des souris surexprimant le gène humain de l'APP.

Un premier aspect de l'invention concerne donc un animal non humain présentant, avantageusement dans son génome, au moins une séquence d'acide nucléique codant pour la préséniline 1 portant les deux mutations correspondant aux mutations M233T et L235P sur la protéine PS1 de souris, à l'état homozygote.

De façon préférentielle la protéine PS1 portant les mutations M233T et L235P est d'origine murine.

De manière particulièrement préférée la protéine préséniline 1 mutée est endogène.

Ainsi un animal selon la présente invention produit avantageusement une protéine comprenant la séquence SEQ ID N°2. Il produit préférentiellement une protéine présentant la séquence SEQ ID N°3. Il comprend avantageusement dans son génome la séquence d'acides nucléiques SEQ ID N°1 ou la séquence SEQ ID N°8.

Les séquences SEQ ID N°1, SEQ ID N°2 et SEQ ID N°8 résultent respectivement des mutations introduites dans les séquences sauvages SEQ ID N°4, SEQ ID N°5 et SEQ ID N°9. La séquence SEQ ID N°5 est celle des résidus 229 à 237 de la protéine préséniline 1 sauvage de souris. La séquence SEQ ID N°9 est celle de l'exon 7 sauvage du gène de la souris codant la protéine préséniline 1, i.e. non mutée.

Avantageusement un animal selon la présente invention coexprime l'APP, préférentiellement l'APP humaine. Un tel gène peut comprendre une ou plusieurs mutations FAD. Ainsi, les mutations dans le gène de l'APP peuvent être l'une d es différentes mutations décrites jusqu'à présent dans la littérature. Les mutations dans le gène de l'APP peuvent être choisies parmi les mutations "Swedish" (S), "London" (L) et "Dutch" (D) seules ou en combinaison.

Ces mutations sont bien décrites dans la littérature et sont caractérisées d'une manière générale par les modifications suivantes :

| Nature et position | Mutation Swedish | Mutation Dutch | Mutation London |
|---|---|---|---|
| par rapport à l'APP770 | K 670 N et M 671 L | E 693 Q et/ou A 692 G | V 717 I |
| par rapport à l'APP751 | K 651 N et M 652 L | E 674 Q et/ou A 673 G | V 698 I |
| par rapport à l'APP695 | K 595 N et M596L | E 618 Q et/ou A 617 G | V 642 I |
| par rapport au peptide A-β (A42) | | E 22 Q et/ou A 21 G | V 46 I |

Sont également comprises par mutation London toutes les substitutions autres que par l'isoleucine qui sont situées en position 717 par référence à l'APP770, telles que par exemple les mutations V 717 G et V 717 F.

Il est entendu que l'APP utilisable dans le cadre de l'invention, peut être sous différentes isoformes et en particulier dans les formes 695, 751 et 770 ou dans une forme tronquée telle que par exemple l'isoforme APP99, excluant la mutation Swedish pour cette dernière.

De manière avantageuse, ledit animal comprend en plus, avantageusement dans son génome, u ne s équence d'acides nucléiques codant pour tout ou une partie du gène codant pour l'APP751. De manière avantageuse la protéine APP751 est d'origine humaine. Elle présente préférentiellement les mutations K670N et M671L (Swedich) et V717I (London).

Dans le cadre de la présente invention, le gène de l'APP est avantageusement placé sous le contrôle de séquences permettant son expression forte dans les neurones et en particulier de séquences promotrices de la transcription tel qu'un promoteur exogène. A titre de séquences promotrices, on peut citer tout particulièrement le promoteur HMG (Gautier et al. (1989), Nucleic Acids Res 17: 20, 8389.), ainsi que le promoteur PDGF (Sasahara et al. (1991), Cell 64, 217-27), le promoteur Thy-1 (Luthi et al (1997), J Neurosci 17, 4688-99) et le promoteur du gène du Prion (Scott et al (1992), Protein Sci 1, 986-97).

Selon une mise en oeuvre particulièrement intéressante de l'invention, le modèle animal comprend le gène de l'APP ayant les mutations S, D et/ou L, placé sous le contrôle du promoteur Thy1.

Ainsi un animal selon la présente invention produit préférentiellement une protéine comprenant la séquence SEQ ID N°7. Il peut présenter la séquence d'acides nucléiques SEQID N°6.

De façon préférentielle, il s'agit d'une souris transgènique issue du croisement entre une souris transgènique ThyAPP (TG53) portant une séquence d'acide nucléique codant la protéine humaine APP751SL et une souris transgènique portant une séquence d'acide nucléique codant la protéine PS1 de souris portant les mutations M233T et L235P.

Les animaux selon la présente invention reproduisent pour la première fois l'une des caractéristiques les plus importantes des maladies neurodégénératives, qui est la perte neuronale de façon précoce.

Ils montrent par ailleurs les autres caractéristiques classiquement décrites de ces pathologies. Les animaux présentent une déposition accélérée des plaques amyloïdes clairement visible dès 2 mois d'age, et de façon marquée à 6 mois.

Ils présentent aussi un rapport des formes Aβ42 sur Aβ total, Aβ42/Aβ supérieur à environ 0,9 et ce dès 2,5 mois. Un tel rapport est très élevé par rapport à celui est décrit dans la littérature pour d'autres souris transgèniques.

La perte neuronale, déjà visible chez des souris âgées de 6 mois, est clairement prononcée à 10 mois.

La PKR (Double strand RNA-dependent Protein Kinase) est une kinase activée par le stress et phosphorylant eIF2, impliquée dans l'apoptose.

La PKR est détectée dans l'hippocampe (la structure ou a lieu la perte neuronale) de souris APPxPS1KI selon l'invention âgées de 10 mois. Elle n'est pas détectée dans l'hippocampe de souris transgéniques APPxPS1M146L âgées de 12 mois chez les lesquelles par ailleurs aucune perte neuronale n'est observée.

Les caractéristiques nouvelles des animaux selon la présente invention en font des modèles d'étude plus complets et représentatifs des troubles observés chez les patients atteints de la maladie d'Alzheimer, que ceux déjà décrits. Ces animaux sont donc particulièrement adaptés pour la mise en évidence des propriétés neuroprotectrices des composés destinés au traitement des maladies neurodégénératives, de façon préférentielle la maladie d'Alzheimer.

De façon préférentielle, les animaux selon la présente invention possèdent les allèles mutants de ps1 à l'état homozygote et ceux de APP à l'état hétérozygote. Cependant, les mêmes caractéristiques du dit animal peuvent être décrites chez un animal possédant l'une des deux allèles ps1 mutée à l'état hétérozygote et celles de APP à l'état hétérozygote, avec néanmoins un phénotype moins marqué ou apparaissant plus tardivement.

Un autre avantage des animaux selon la présente invention est que le taux de protéine PS1 mutée exprimée par cette souris transgènique est équivalent au taux de protéine PS1 endogène normalement exprimée par une souris normale (non transgènique), exprimant une PS1 non mutée. Cette caractéristique en fait un modèle d'étude avantageux - sans surexpression de la protéine PS1- pour la mise en évidence de composés destinés au traitement des maladies neurodégénératives.

Ces composés peuvent être notamment des composés ayant une action sur la régulation du gène PS1 au niveau transcriptionnel, post-transcriptionnel, traductionnel, post-traductionnel, ou sur la protéine PS1 elle-même en modifiant ou régulant une ou plusieurs de ses propriétés, ou ayant une action semblable sur les partenaires d'interaction ou les cibles de la protéine PS1, ou encore de composées ayant une action sur la régulation de l'APP et de façon plus large toutes molécules en aval des signaux initiés par PS 1 et APP au cour du processus neurodégénératif.

Dans le cadre de la présente invention, les animaux sont avantageusement des mammifères, tels que des rongeurs. En particulier il peut s'agir d'une souris, d'un rat ou d'un lapin.

Les souris et les constructions permettant leur obtention sont obtenues par des méthodes connues de l'homme du métier.

Ils peuvent être obtenus selon les techniques classiques de transgenèse. A titre d'exemple illustrant l'un des procédés de transgenèse, on peut citer la méthode d'électroporation d'une construction gènique contenant les gènes modifiés dans des cellules souches embryonnaires de souris et, après sélection, transfert des cellules porteuses de l'évènement génétique désiré dans un blastocyste receveur, tel que décrit dans les exemples. A cet égard, les animaux PS1 mutés selon l'invention sont obtenus par électroporation d'une cassette d'expression comprenant un acide nucléique. De manière préférentielle, cet acide nucléique est un ADN qui peut être un ADN génomique (ADNg) ou un ADN complémentaire (ADNc).

La modification du génome peut résulter d'une altération ou une modification d'un ou plusieurs gènes par « knock-in ». Cette modification peut être due à l'action d'agents altérants ou mutagènes classiques ou bien peut-être effectuée par mutagenèse dirigée. Dans la présente invention, en ce qui concerne le gène ps1 muté, il s'agit préférentiellement d'une recombinaison homologue avec un vecteur de ciblage portant le transgène préalablement muté par mutagenèse dirigée telle que décrit dans les exemples qui suivent.

Les animaux exprimant la protéine APP mutée sont obtenus par micro-injection d'une construction gènique dans le noyau d'un zygote.

Les animaux double transgèniques sont obtenues par croisement des animaux ps1 mutés et des animaux APP mutés.

Les animaux selon la présente invention peuvent avantageusement être utilisés pour la mise en évidence des propriétés neuroprotectrices des composés destinés au traitement des maladies neurodégénératives, et de préférence la maladie d'Alzheimer. Ces composés peuvent être des molécules chimiques, des molécules peptidiques ou protéiques, des anticorps, des molécules chimériques ainsi que des ARNs antisens ou des ribosimes. Les composés mis en évidence peuvent être utilisés comme médicaments, tels quels ou en association avec un véhicule pharmaceutiquement acceptable afin d'obtenir une composition pharmaceutique. Il peut s'agir en particulier de solutions salines (phosphate monosodique, disodique, chlorure de sodium, potassium, calcium ou magnésium, etc., ou des mélanges de tels sels), stériles, isotoniques, ou de compositions sèches, notamment lyophilisées, qui par addition selon les cas d'eau stérilisée ou de sérum physiologique, permettent la constitution de solutés injectables. Les injections pouvant être réalisées par voie stéréotaxique, topique, orale, parentérale, intranasale, intraveineuse, intramusculaire, sous-cutanée, intraoculaire, transdermique, etc.

Un autre objet de l'invention concerne donc un procédé de mise en évidence de composés destinés au traitement des maladies neurodégénératives comprenant au moins les étapes suivantes :
- administration du composé ou d'un mélange de composés à tester à des animaux selon la présente invention, et
- observation de l'évolution d'un ou plusieurs marqueurs caractéristiques reproduisant la neuropathologie observée chez l'homme.

Un autre objet de l'invention concerne un procédé de mise en évidence de composés destinés au traitement des maladies neurodégénératives comprenant au moins les étapes suivantes :
- mise en contact de cellules extraites des animaux selon la présente invention avec un composé ou un mélange de composés, et
- mesure du ou des effet(s) des composés au niveau des cellules entières, dans des homogénats de cellules ou sur une fraction subcellulaire.

Un autre objet de l'invention concerne tout produit biologique issu d'un des deux animaux de l'invention, ainsi que leurs utilisations pour la mise en évidence de composés destinés au traitement des maladies neurodégénératives, de préférence la maladie d'Alzheimer. On entend notamment par « produit biologique », des cellules, extraits protéiques, ADN, ARN, ou encore des anticorps.

Ainsi, la présente invention a pour objet des cellules ou lignées cellulaires dérivées d'un animal tel que décrit ci-dessus, en particulier des cellules souches embryonnaires.

La présente invention a en outre pour objet une protéine PS1 de souris portant les mutations des acides aminés M en T, et L en P respectivement aux positions 233 et 235. De manière avantageuse une telle protéine comprend la séquence SEQ ID N°2. Préférentiellement elle présente la séquence SEQ ID N°3.

Un autre objet de la présente invention est un acide nucléique codant la protéine PS1 de souris portant les mutations des acides aminés M en T, et L en P respectivement aux positions 233 et 235.

De manière a vantageuse un tel acide nucléique selon la revendication comprend la séquence SEQ ID N°1 ou la séquence SEQ ID N°8.

La présente invention a en outre pour objet les séquences complémentaires de ces acides nucléiques et des vecteurs comprenant ces acides nucléiques ou leurs séquences complémentaires.

Un autre aspect de l'invention concerne l'utilisation de ces protéines pour la mise en évidence des propriétés neuroprotectrices des composés destinés au traitement des maladies neurodégénératives.

La présente invention est illustrée par les exemples qui suivent, sans pour autant qu'elle soit limitée à ces seuls exemples.

Dans ces exemples, les résultats décrits démontrent l'avantage des souris PS1KI et supportent clairement l'utilisation préférentielle du modèle PS1KIxAPP dans les stratégies thérapeutiques car il a l'avantage de représenter les principales caractéristiques des maladies neurodégénératives connues à ce jour.

### LEGENDES DES FIGURES

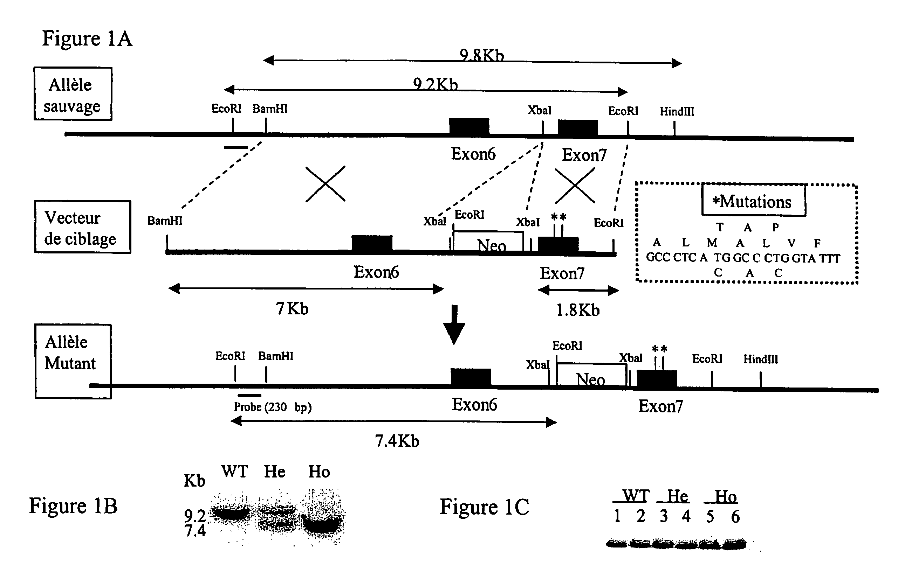

**Figure 1** **A** : Représentation schématique de la structure du gene ps1 murin et des principaux sites de restriction autour de l'exon 7 sauvage (ligne supérieure) et du vecteur de ciblage utilisé (ligne du milieu). Les changements de base nucléotidiques pour générer les mutations de codons M233T et L235P mutations dans l'exon 7 (*) sont représentés dans la cartouche en pointillés. L'allèle muté PS1KI contenant la cassette de résistance néomycine (Neo) est représenté sur la ligne inférieure. La position de la sonde de 230-bp utilisée pour l'identification des nouveaux-nés est aussi indiquée.
**Figure 1** **B** : Southern-blot en utilisant la sonde de 230-bp pour distinguer les allèles sauvages WT (bande à 9,2 kb), et PS1KI hétérozygotes (He, double bande) et homozygotes (Ho, bande à 7.4 kb) chez différentes souris.
**Figure 1** **C** : Immunotransfert du fragment C-terminal de PS1 montrant que les niveaux d'expression de la protéine PS1 ne sont pas altérés par la présence des mutations de l'allèle PS1KI.

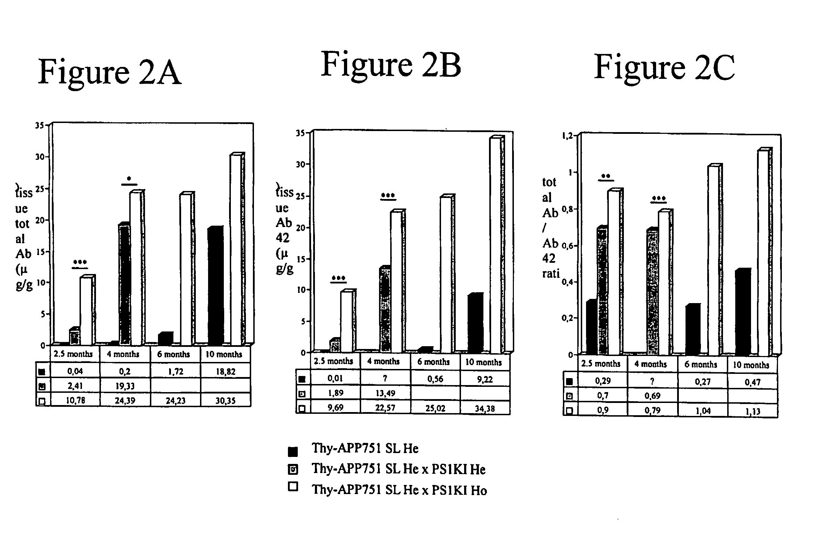

**Figures 2** **A, 2B et 2C :** Quantification, respectivement, de l'Aβtotal de l'Aβ42 et du rapport Aβtotal/Aβ42, à 2,5, 4, 6 et 10 mois d'âge.

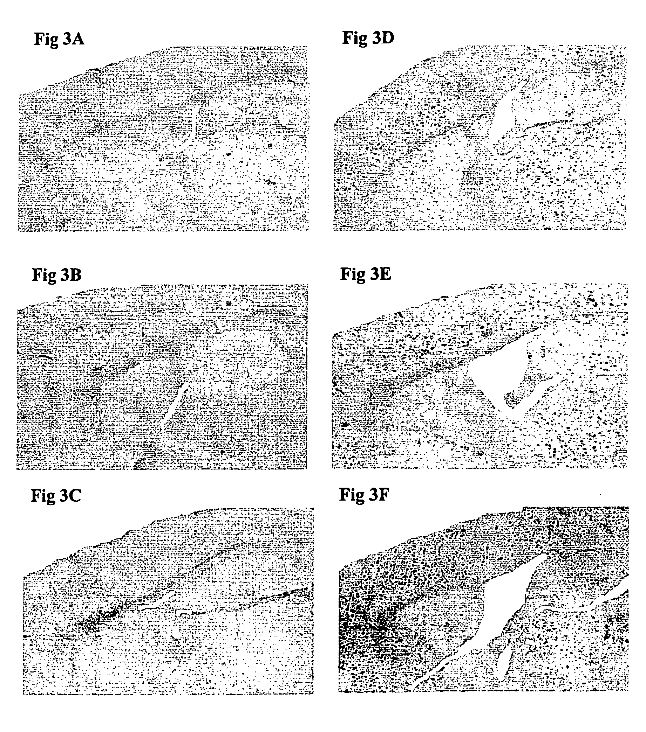

**Figure 3** **:** Accélération du processus de déposition du peptide Aβ chez les souris APP751SLxPS1KI Ho. Planche illustrative de la distribution régionale des dépôts extracellulaires du peptide Aβ dans le cerveau à 6 mois. Les images représentent l'immunomarquage Aβ (Ac 4G8) chez 3 souris APP751SL (Fig 3A, 3B, 3C) et 3 souris APP751SLxPS1KI Ho (Fig 3D, 3E, 3F). L'immunomarquage met en évidence l'apparition à 6 mois des premiers dépôts encore rares dans le cortex (Cx) et l'hippocampe (Hp) des souris APP751SL. Comparativement, chez les souris APP751SLxPS1KI Ho du même âge, le nombre de dépôts est largement augmenté dans ces régions. A noter que, chez ces souris, des dépôts sont déjà présents en quantité notable dans le thalamus (T).

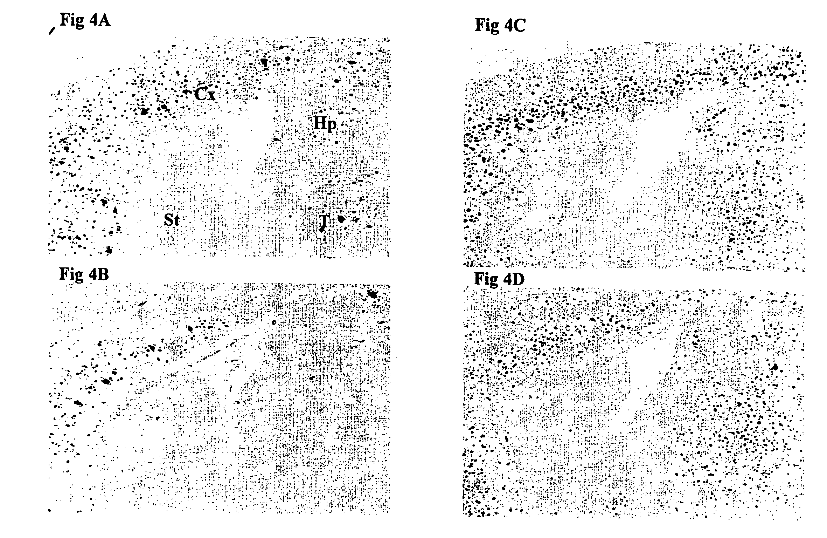

**Figure 4** **:** Progression avec l'âge du processus de déposition du peptide Aβ. Planche illustrative de la distribution régionale des dépôts Aβ dans le cerveau à 10 mois. Les images correspondent à 2 souris APP751SL (Fig 4A, 4B) et 2 souris APP751SLxPS1KI Ho (Fig. 4C, 4D). Chez les souris APP75 1 SL, l'immunomarquage met en évidence une augmentation importante du nombre et de la taille des dépôts dans le cortex (Cx) et l'hippocampe (Hp) à 10 mois comparativement à 6 mois d'âge et l'apparition des premiers dépôts dans le thalamus (voir figure 3). La densité et la taille des dépôts sont également plus importantes à 10 mois dans le cortex, l'hippocampe et le thalamus des souris APP751SLxPS1KI Ho. A noter que, chez ces souris, des dépôts en petit nombre peuvent être détectés dans le striatum (St).

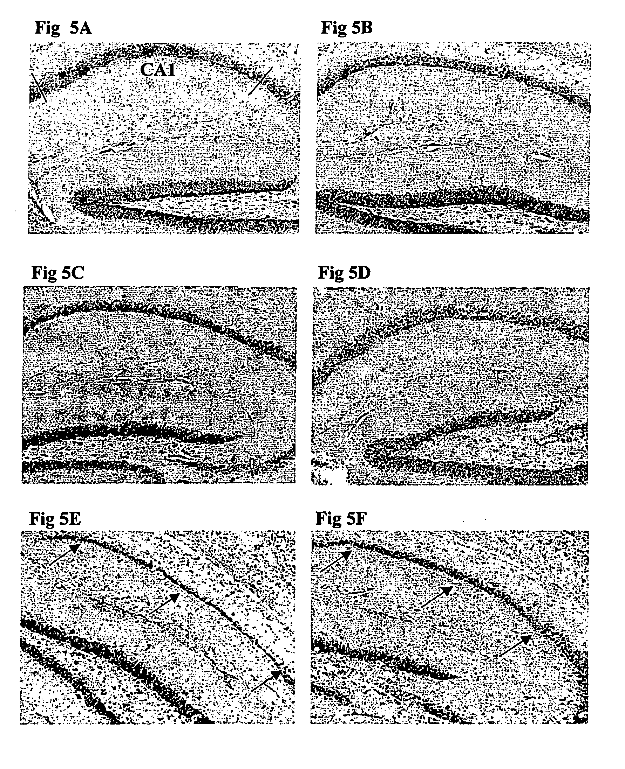

**Figure 5** : Processus de mort neuronale dans CA1 chez les souris APP751SLxPS1KI Ho. Planche illustrative de l'atteinte des neurones pyramidaux dans l'hippocampe de souris APP751SLxPS1KI Ho âgées de 10 mois. Les images représentent la coloration violet de Crésyl, à faible grossissement dans l'hippocampe, chez 2 souris PS1KI Ho (Fig. 5A, 5B), 2 souris APP751SL (Fig. 5C, 5D) et 2 souris APP751SLxPS1KI Ho (Fig. 5E, 5F). La densité et l'épaisseur des couches des cellules pyramidales dans l'hippocampe sont qualitativement comparables chez les souris APP751SL et les souris PS1KI Ho âgées de 10 mois. En revanche, au même âge, elles sont nettement diminuées chez les souris APP751SL x PS1KI Ho, en particulier dans la couche 1 de la Corne d'Ammon (CA1). A noter que le nombre de petites cellules colorées en bleu (cellules de type glial) apparaît augmenté dans l'hippocampe des souris APP75 1 SL x PS1KI Ho.

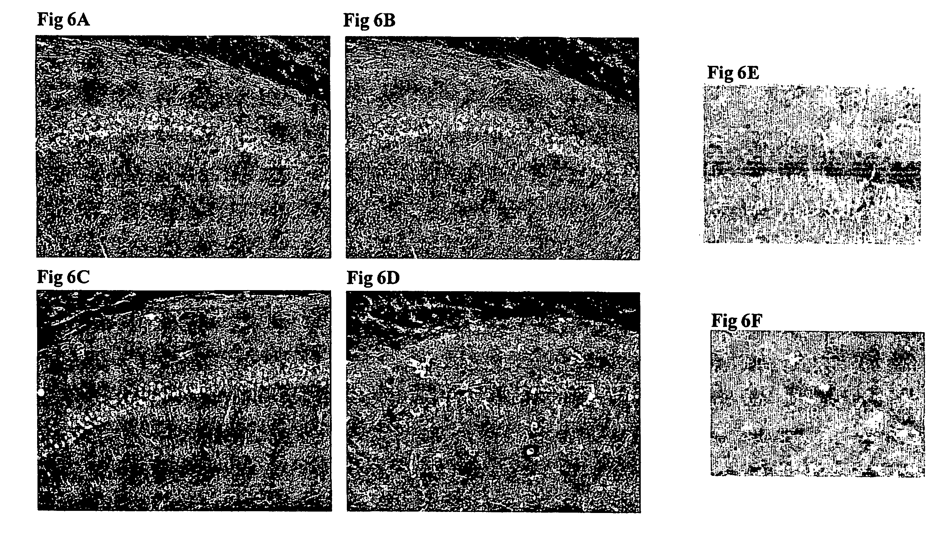

**Figure 6** : Processus de mort neuronale dans CA1 chez les souris APP751SLxPSIKI Ho. Planche illustrative de l'atteinte neuronale dans CA1 à 10 mois via l'utilisation d'autres marqueurs neuronaux, le vert de méthyle et l'immunomarquage BIP. Les images représentent la coloration vert de méthyle, à fort grossissement dans CA1, chez une souris non transgénique (Fig 6A), une souris PKS1KI Ho (Fig 6B), une souris APP751SL (Fig 6C) et une souris APP751SLxPS1KI Ho (Fig 6D). Elles représentent l'immunomarquage BIP à fort grossissement dans CA1, chez une souris PS1KI Ho (Fig 6 E) et une souris APP751SLxPS1KI Ho (Fig 6F). Comparativement aux souris non transgènique, PS1 KI Ho et APP751SL, le nombre de cellules neuronales colorées au vert de méthyl est nettement diminué dans la région CA1 de la souris APP751SLxPS1KI Ho. A noter la détection de cellules type glial colorées en nombre important dans le parenchyme hippocampal de cette souris double transgènique. L'immunomarquage BIP confirme également la perte de neurones pyramidaux importante dans CA1 chez la souris APP751SLxPS1KI Ho âgée de 10 mois.

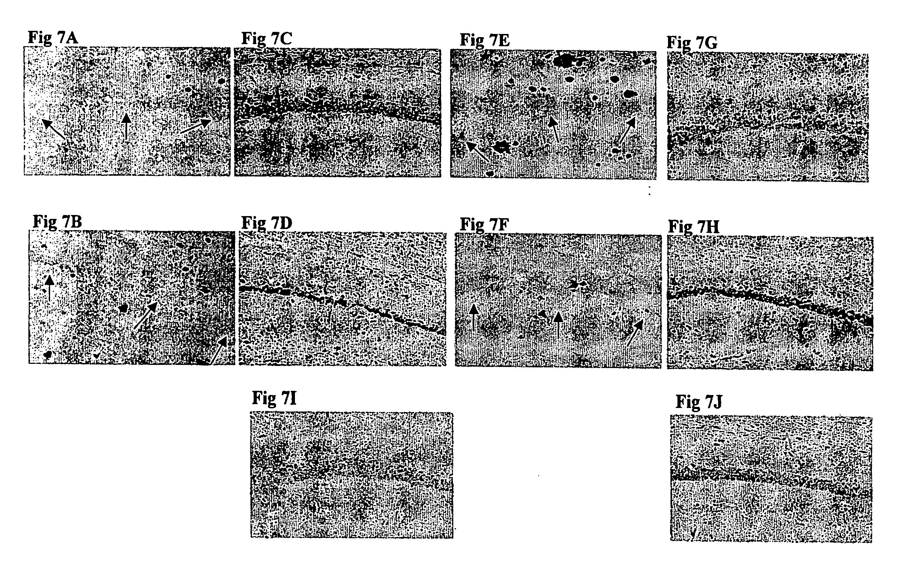

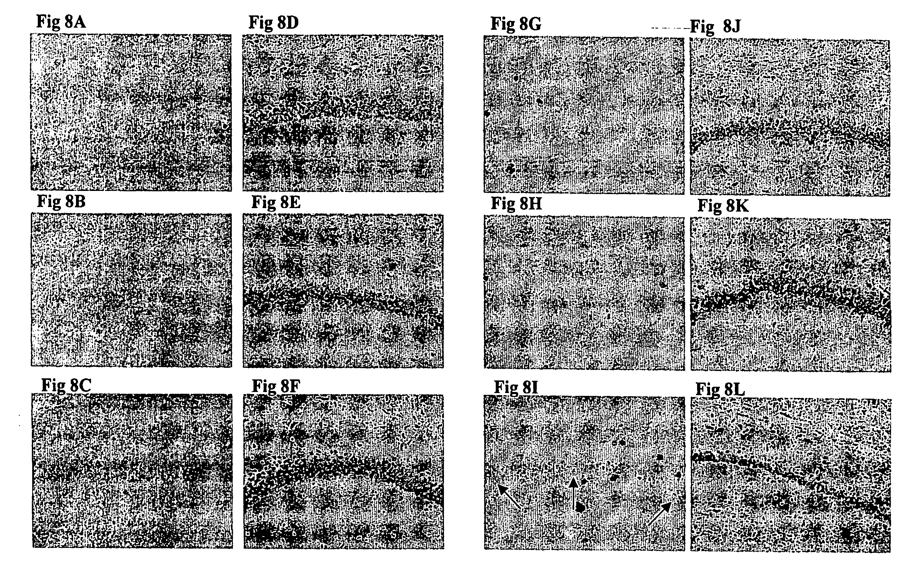

**Figure 7****:** Mort neuronale dans CA1 et déposition intracellulaire du peptide Aβ. Planche illustrative des deux processus pathologiques, l'atteinte neuronale et l'accumulation intracérébrale anormale du peptide Aβ à 10 mois. Les images représentent, à fort grossissement dans CA1, l'immunomarquage Aβ chez 2 souris APP751 (Fig. 7A, 7B) et 2 souris APP751SLxPS1KI Ho (Fig 7E, 7F). Elles représentent, à fort grossissement dans CA1, la coloration violet de Crésyl chez les souris APP751 (Fig. 7C, 7D), les souris APP751SLxPS1KI Ho (Fig. 7G, 7H) et 2 souris PS1KI Ho (7I, 7J). A 10 mois d'âge, aussi bien chez les souris simples APP751SL que chez les doubles APP751SL x PS1KI Ho, les dépôts extracellulaires d'Aβ sont observés majoritairement de part et d'autre de la couche de neurones dans CA1. En revanche, dans CA1 (caractérisée par une atteinte neuronale prononcée tout le long de la couche chez les souris APP751SLxPS1KI Ho, Fig. 7C, 7D), l'immunomarquage Aβ d'aspect granulaire (correspondant à l'accumulation intraneuronale anormale du peptide Aβ, voir flèches) apparaît plus intense chez les souris APP751SL x PS1KI Ho. Ceci est vrai aussi à 6 mois d'âge (voir Figure 8).
**Figure 8** **:** Mise en place précoce du processus de mort neuronale dans CA1 chez les APP751SLxPS1KI Ho. Planche illustrative de la région CA1 de l'hippocampe à 6 mois. Les images représentent, à fort grossissement dans CA1, l'immunomarquage Aβ chez 3 souris APP751 (8A, 8B, 8C) et 3 souris APP751SLxPS1KI Ho (Fig. 8G, 8H, 8I). Elles représentent la coloration violet de Crésyl chez les souris APP751 (Fig. 8D, 8E, 8F) et les souris APP751SLxPS1KI Ho (8J, 8K, 8L). A 6 mois, la région CA1 de l'hippocampe, chez une souris APP751SLxPS1KI Ho, est caractérisée par un nombre de dépôts extracellulaires d'Aβ déjà important (Fig 8I), un marquage granulaire intracellulaire d'Aβ intense (voir flèches) et une perte de neurones colorés au violet de Crésyl associée à une augmentation du nombre de cellules type glial (Fig 8L). Pour les 2 autres souris APP751SLxPS1KI Ho, la couche de neurones CA1 colorée au violet de Crésyl apparaît peu (Fig 8J) ou pas (Fig 8K) désorganisée. A noter que pour ces deux souris, l'immunomarquage Aβ intracellulaire apparaît moins intense et plus diffus (FiG 8G, 8H) que chez la 3° souris (Fi 8I).

### EXEMPLES

**Exemple 1 : Construction du vecteur de ciblage portant les mutations M233T et L235P** Le but a été d'introduire deux mutations dans l'exon7 du gène PS1 de la souris conduisant à l'altération des résidus M233 en T et L235 en P. Les deux nouveaux codons correspondent à des mutations identifiées dans des patients Alzheimer à début précoce (FAD).

Une lignée de souris PS1 knock-in (PS1KI) a été générée en utilisant une stratégie de mutagénèse en 2 étapes similaire à celle décrite dans Kwok et al (1997 Neuroreport 8; 157-42) et Champion et al. (1996, Neuroreport 7, 1582-4).

La stratégie a visé à la construction d'un vecteur de ciblage porteur de changements d'acides nucléiques dans les codons 233 et 235 du gène murin *ps*1 (voir Fig1A).

Succinctement, un fragment génomique de 17-Kb du gène souris PS1 a été isolé par criblage d'une banque d'ADN génomique de souris 129SvJ construite dans un bactériophage lambda (Stratagene, catalogue # 946313). L'analyse par digestion avec des enzymes de restriction, le séquençage et la comparaison avec les séquences partielles du gène murin PS1 disponibles (Mitsuda et al. 1997, JBC 272, 23489-97), ont indiqué que ce fragment contenait la région intron5 à exon 11 du gène souris *PS1*. Un sous-fragment *BamH*I-*Hind*III de 9.8-Kb contenant une partie de l'intron 5, l'exon 6, l'intron 6, l'exon 7 et une partie de l'intron 7, a été sous-clonée dans le plasmide pGEM-11Zf (+) (Promega, France) (Fig.lA). La mutagénèse des 2 codons a été réalisée en utilisant le kit Gene Editor (Promega ) sur le fragment d'ADN contenant l'exon 7 et a été confirmée par séquençage nucléotidique.

Le bras long (5') du vecteur de recombinaison homologue a été obtenu par clonage du fragment *BamH*I-*Xba*I de 7-Kb contenant l'exon 6. Le bras court (3') al ui é té généré par sous-clonage du fragment *Xba*I-*EcoR*I de 1.8-Kb contenant l'exon 7 mutagénéisé. Une cassette de sélection positive (pMCI-Neo cassette) a été introduite dans le site *Xba*I situé dans l'intron 6 à la position -470 bp en 5' de l'exon 7 (voir Fig1A).

### Exemple 2 : Obtention de cellules ES comprenant PS1KI

Le vecteur de ciblage, décrit dans l'exemple 1, a été linéarisé par digestion avec *Not*I et électroporé dans la lignée de cellules souche embryonnaire (ES) CK35 fournie par le Dr. Charles Babinet, Institut Pasteur Paris, France.

Les cellules ont été cultivées comme précédemment décrit (W.Wurtz and A. Joyner, Gene Targeting: A Practical Approach by Alexandra L. Joyner (Editor). Oxford University Press; 2nd edition (February 15, 2000)).

430 clones cellulaires susceptibles de porter la recombinaison homologue ont été sélectionnés en présence de G418. L'ADN génomique de ces clones a été analysé par Southem-blot comme précédemment décrit (Sambrook, Fritsch and Maniatis, Molecular Cloning ; A Laboratory Manual, Cold String Harbor Laboratory Press, 2nd edition, 1989) en utilisant une sonde PS1 située en dehors du domaine de recombinaison (Fig1A) du bras long. Quatre clones cellulaires porteurs des mutations souhaitées dans le gène *PS1* ont a insi p u être identifiés. C es clones cellulaires ont servi à établir une lignée de souris transgèniques PS1KI.

### Exemple 3 : Construction de la lignée de souris PS1KI

Le clone 18C5 a été injecté dans des blastocytes de souris C57B1/6.

Cinq des souris chimériques obtenues ont montré une transmission de l'allèle mutant *ps*1 à la lignée germinale (et donc à leur descendance).

A partir de ces fondateurs, la lignée de souris PS1KI a été établie sur fond génétique pur 129SV et sur fond mixte 129SV-C57Bl/6.

La présence de l'allèle muté PS1KI à l'état hétérozygote (He) ou homozygote (Ho)-a été déterminée par Southern-blots avec la sonde *ps*1 de 230-bp (Fig.1B). Les souris mutantes sont viables et fertiles.

### Exemple 4 : Dosage de PS1 dans la lignée PS1KI

Après euthanasie, le cerveau des souris a été prélevé et pesé. Un hémisphère a été conservé pour l'immunohistochimie (post-fixation) et l'autre a été congelé puis homogénéisé individuellement sur glace à l'aide d'un Potter dans 2 mL d'une solution tampon: 0,32 M sucrose, Tris-HCl 4 mM, pH 7,4 contenant un cocktail d'inhibiteurs de protéases (Complete^{™}, Roche Diagnostics). La concentration en protéine a été déterminée par la méthode BCA (Pierce). L'homogénat a été conservé à -80°C.

Pour la détection de PS1, 25 µg d'extrait protéique de cerveau ont été incubés à 56°C pendant 20 min dans du tampon de dépôt Laemmli contenant de l'urée 8M et du dithiothreitol 50 mM. Les protéines ont été fractionnées par électrophorèse sur gel NuPAGE 4-12% Bis-Tris polyacrylamide (SDS-PAGE) en tampon MES (acide 2-(N-morpholino) éthanesulfonique). Après transfert des protéines sur filtre de nitrocellulose (Amersham, France), le filtre a été chauffé dans du PBS pendant 5 min afin d'augmenter la sensibilité et immédiatement saturé avec 5% (w/V) de lait écrémé en poudre dans un tampon PBST (PBS, 0.05% (V/V) Tween 20) pendant 1h et incubé la nuit à 4°C avec l'anticorps primaire en tampon PBST seul. La liaison de l'anticorps a été détecté avec un anticorps -anti IgG (anti-souris) conjugué à la peroxidase de Raifort (Amersham, France) à la dilution de 1/10,000 en PBST suivi d'un système de détection par chimioluminescence (Amersham, France) selon les instructions du fabricant. Pour la détection de PS1, l'anticorps primaire MAB1563 (Chemicon, USA) a été utilisé à la dilution 1/10000. Pour l'analyse semi-quantitative, les signaux de luminescence ont été digitalisés avec une camera CCD GeneGnome 16 bits (Syngene, Cambridge, Angleterre) et analysés avec le logiciel Genetools (Syngene). La linéarité du signal a été vérifiée grâce à des courbes standards établies avec des échantillons de 2.5 à 10 µg d'homogénat par piste.

Cette analyse par immunotransfert a permis de déterminer que les niveaux d'expression du fragment C-term de PS1 muté restent normaux et ne sont pas diminués dans la souris PS1KI233/235 (Fig1C).

### Exemple 5 : Obtention de la lignée PS1KIxAPP par croisement des lignées PS1KI et APP

Les souris PS1KI (décrites dans les exemples 1 à 4) ont été croisées avec une lignée de souris transgèniques surexprimant la forme humaine du cDNA APP₇₅₁ portant les mutations FAD Swedish (mutation K670N; M671L) et London (V717I) sous le contrôle du promoteur Thy-1. Les souris surexprimant la forme humaine du cDNA APP₇₅₁ portant les mutations ont été obtenues comme décrit dans la demande de brevet WO O1/20977.

Dans toutes les expériences suivantes, les souris de même fonds génétique ont été utilisées pour minimiser tout effet dû à des variations de fond génétique.

### Exemple 6 : Dosage du peptide amyloïde Aβ total et Aβ42 par la méthode d'immunoélectrochimioluminescence

Pour la quantification du pool global d'Aβ dans le cerveau (formes solubles et formes agrégées ou insolubles), des aliquotes d'homogénat de cerveau ont été traités avec 2 volumes d'une solution de 9M d'hydrochlorate de guanidine (GH) en 50 m MT ris pH7.4. Les homogénats ont été mélangés pendant 1h avec 3 périodes de sonication de 15-min suivies d'une centrifugation à 50 000g à 4°C pendant 2h. Les extraits guanidine ont été dilués au 1/20eme en tampon 20 mM Tris-HCl, pH 7.6, 150 mM NaCl, 0.5% BSA (w/v) and 0.05% Tween 20 (w/v). La concentration du peptide Aβ dans les fractions a ensuite été déterminée par immunoélectrochimioluminescence (Yang et al., 1994, Biotechnology (NY) 12(2), 193-194) à l'aide de 2 anticorps monoclonaux de souris anti-peptide Aβ (4G8 et 6E10) et du lecteur Origen M8 Analyzer (IGEN Europe Inc. Oxford) suivant un protocole modifié d'après Khorkova et al. (J. Neurosci. Methods 82, 159-166 (1998)).

L'anticorps monoclonal 4G8 (Senetek PLC), reconnaissant l'épitope résidus 17-24 du peptide Aβ, est ruthénylé grâce à l'ester TAG-NHS suivant le protocole du fournisseur (IGEN Europe Inc., Oxford). Ru-4G8 et l'anticorps biotinylé 6E10, épitope 1-10 du peptide Aβ (Senetek PLC) sont mis en présence de la fraction soluble de cerveau et les complexes tripartites Ru-4G8/Aβ/6E10-biot quantifiés par le lecteur Origen. Une g amme de peptide synthétique A β (Bachem) est utilisée pour calibrer chaque expérience. Le taux de peptide Aβ est calculé en nanogrammes par g de poids initial de tissu cérébral.

Pour mesurer spécifiquement les formes de peptide Aβ se terminant en position 42 (Aβ42), l'anticorps 6E10 était remplacé par l'anticorps monoclonal 22F9 qui se fixe spécifiquement à l'extrémité C-terminale Aβ42 (Wirths et al., 2002, Brain Pathol. 12, 275-286).

En conclusion, la présence de du gène ps1 knock-in (PS1-KI) conduit à :
- Une accélération de l'accumulation de Aβ (Fig2A) et Aβ42 (Fig2B) dans le cerveau avec un effet encore plus prononcé lorsque l'allèle PS1KI est présent à l'état homozygote (effet gène-dosage). L'effet de PS1KI(Ho) est plus accentué qu'avec la souris transgènique surexprimant PS1M146L précédemment décrite dans la demande WO O1/20977.
- Une augmentation massive de la proportion de peptide Aβ présentant une extrémité β42, qui représente la grande majorité de l'Aβ lorsque la mutation PS1KI est à l'état homozygote, comme le montre la figure 2C (ratio Aβ42/Aβ total égal à 0.92, à 2.5 mois d'âge, vs 0.25 en absence de PS1KI et une valeur intermédiaire 0.70 en présence d'une seule allèle PS1KI: effet gène-dosage). Il est reconnu dans la littérature que les espèces de peptide Aβ se terminant à l'extrémité β42 représentent les formes les plus pathologiques du peptide. La lignée PS1KIxAPP représente donc un modèle particulièrement enrichi en formes pathologiques.

### Exemple 7 : Analyse des dépôts du peptide Aβ par immunohistochimie

Pour les expériences d'immunohistochimie/histologie, après prélèvement puis postfixation dans du paraformaldéhyde 4 %, les hémicerveaux sont cryoprotégés pendant 1 nuit à 4°C, dans du tampon phosphate de sodium 0.2M (NaH₂PO₄,2H₂O / Na₂HPO₄, 12H₂O, pH 7.4) contenant du saccharose à 20% (P/V). Ils sont ensuite congelés pendant 1 min dans de l'isopentane maintenu à une température de -30°C dans de la carboglace. Des coupes de 25µm d'épaisseur, réalisées dans un cryostat thermostaté à -30°C (LEICA CM3000) sont finalement placées dans un tampon PBS 0.02M puis conservées à 4°C.

Sur ces coupes, la détection immunoenzymatique du peptide Aβ, a été réalisée grâce au système de révélation impliquant la formation de complexes avidine-biotine-peroxydase (ABC) dans lequel la peroxidase de Raifort couplée à l'avidine est biotinylée. Brièvement, après une incubation de 30 min dans du tampon de blocage (sérum normal de chèvre (Chemicon) à 10 % dans du PBS contenant 0,1 % de triton (Sigma), les coupes de cerveaux sont mises en présence d'une solution de H₂O₂ à 0.3 % afin d'éliminer les endopéroxidases présentes dans le tissu. Puis ces coupes sont incubées dans la solution d'anticorps primaire contenant 0.3 % de triton et 2 % de sérum normal (toute la nuit à 4°C). L'anticorps primaire anti-Aβ (4G8, Senetek) (anticorps monoclonal dirigé contre le résidu 17-24 du peptide Aβ) utilisé est biotinylé. Après rinçages, les coupes ont donc été mises directement en présence du complexe ABC, pendant 1 heure selon les instructions du fabricant (Kit ABC Vectastin, Laboratoires Vector, Burlingame, CA). La 3-3'-diaminobenzydine a été utilisée comme chromogène pour l'enzyme peroxidase.

Ainsi, l'accélération de l'accumulation anormale du peptide Aβ dans le cerveau des souris doubles transgèniques APP751SLxPS1KI Ho, détectée précédemment par des tests biochimiques sur homogénats d'hémicerveaux, a été confirmée par immunohistochimie. En effet, l'analyse microscopique de l'immunomarquage Aβ obtenu sur coupe d'hémicerveaux a démontré l'existence d'un processus de déposition du peptide Aβ accéléré dans le parenchyme cérébral de ces souris. En effet, tandis que les premiers dépôts apparaissent dans le cortex et l'hippocampe vers 6 mois d'âge chez les souris APP751SL (Fig. 3), ils peuvent être détectés dès l'âge de 2 m ois chez les doubles APP751SLxPS1KI à l'état homozygote. Comparativement aux simples transgèniques APP751SL, chez les doubles transgèniques (APP751SLxPS1KI Ho) âgées de 6 mois, la densité des dépôts d'Aβ est nettement plus importante dans l'hippocampe et le cortex. De plus, la distribution des dépôts est plus large; en particulier des dépôts sont déjà détectés dans le thalamus et aussi le pons (Fig.3).

Avec l'âge, en particulier à 10 mois, la densité et également la taille des dépôts sont augmentées dans le cerveau des souris simples transgèniques APP751SL (Fig 4). La distribution de ces dépôts est également plus large puisqu'ils sont présents dans le thalamus. Chez les doubles transgèniques APP751SLxPS1KI Ho âgées de 10 mois, une progression similaire du processus de déposition du peptide Aβ est observée dans l'hippocampe, le cortex, le thalamus et le pons. Les premiers dépôts peuvent être détectés en nombre limité dans le striatum (Fig. 4). En revanche, le cervelet demeure épargné par le processus de déposition d'Av. A noter que dans le cerveau des souris PS1KI Ho âgées de 10 mois (n = 4), aucun dépôt du peptide Aβ n'est détecté.

### Exemple 8 : Analyse de la perte neuronale par histologie et immunohistochimie

La présence d'une proportion très importante de peptide Aβ42 pathologique, a conduit à analyser si dans la lignée APP751SLxPS1KI Ho, outre l'accélération du processus de déposition du peptide Aβ, une perte neuronale se développe avec l'âge. Pour ceci, 3 types de colorations permettant de visualiser la disparition de cellules neuronales sur coupes de tissu cérébral ont été réalisées: a) l'histologie au violet de crésyl qui colore les corps de Nissl (des organites cytoplasmiques associés aux ribosomes du réticulum endoplasmique granuleux) et permet la mise en évidence sur coupes de cerveau de l'ensemble des cellules neuronales et gliales; b) l'histologie au vert de méthyle qui colore l'ADN de toutes les cellules; c) l'immunohistochimie au BIP qui révèle l'expression dans les cellules d'une protéine chaperon résidente du réticulum endoplasmique.

Pour la coloration au violet de crésyl, les coupes de tissu cérébral sont montées sur lames gélatinées puis incubées 10 minutes dans une solution de violet de crésyl (C 1791, Sigma) à 0.5 % dans l'eau distillée. Après un rinçage en milieu acide, les coupes sont finalement déshydratées.

Pour la coloration au vert de méthyle, les coupes sont montées sur lames gélatinées, incubées pendant 10 minutes dans une solution de vert de méthyle (M5015 de sigma) à 1 % dans l'eau distillée, rincées puis déshydratées.

Pour l'immunohistochimie BIP (anticorps polyclonal, SPA-826, Stressgen), le protocole est identique à celui appliqué pour l'immunohistochimie du peptide Aβ (voir ci-dessus) exceptée l'incubation supplémentaire (1h, à température ambiante) des coupes dans une solution d'anticorps secondaire biotinylé (anticorps anti-IgG de lapin fait chez la chèvre, Vector) avant leur incubation dans le complexe ABC.

L'analyse microscopique a mis en évidence, via l'utilisation de différents marqueurs histologiques/immunohistochimique, une diminution de l'épaisseur de la couche des cellules pyramidales de l'hippocampe, en particulier de CA1, dans le cerveau des souris APP751SLxPS1KI Ho (n = 3/3) (Figures 5 et 6). Cette diminution indique l'existence d'un processus de mort neuronale déjà bien installé à l'âge de 10 mois. A 6 mois, la mort neuronale est présente dans le cerveau d'1/3 souris suggérant la mise en place précoce d'un processus neurotoxique (Figure 8). L'analyse en parallèle dans l'hippocampe, et en particulier dans CA1, des 2 processus pathologiques que sont l'accumulation anormale du peptide Aβ dans le cerveau et l'atteinte neuronale, suggère un rôle plus probable dans le processus neurotoxique de l'accumulation intracellulaire d'Aβ (phénomène déjà décrit chez les souris thy1APP751SLxPS1 M146L) que de son accumulation en dépôts extracellulaires (Figure 7). En effet, les neurones encore présents dans CA1 présentent une expression anormalement forte du peptide Aβ. De plus, l'atteinte neuronale dans CA1 est clairement présente dans des régions dépourvues de dépôts extracellulaires. A noter l'existence d'un probable effet gène-dosage dans le processus de mort neuronale dans CA1. Une atteinte neuronale a été en effet retrouvée chez des souris APP751SLxPS1KI très âgées (> 15 mois) n'ayant qu'un allèle PS1KI.

## Revendications

1. Animal non humain présentant une séquence d'acides nucléiques codant pour une protéine préséniline 1 portant à l'état homozygote les mutations correspondant aux mutations M233T et L235P sur la protéine PS1 de souris.

2. Animal selon la revendication 1 comprenant en plus une séquence d'acides nucléiques codant pour tout ou une partie du gène codant pour l'APP.

3. Animal selon l'une des revendications 1 et 2 **caractérisé en ce qu'**il exprime une quantité de PS1 mutée comparable à la quantité de PS1 endogène d'un animal exprimant une PS1 non mutée.

4. Animal selon l'une des revendications 1 à 3 **caractérisé en ce que** la protéine préséniline 1 portant les mutations M233T et L235P est d'origine murine.

5. Animal selon l'une des revendications 2 à 4 **caractérisé en ce que** la protéine APP est l'APP751 et est d'origine humaine.

6. Animal selon l'une des revendications 2 à 5 **caractérisé en ce que** la protéine APP751 est d'origine humaine et présente les mutations Swedish et London.

7. Animal selon l'une des revendications 1 à 6 **caractérisé en ce qu'**il est un rongeur.

8. Animal selon l'une des revendications 1 à 7 **caractérisé en ce qu'**il est une souris, un rat ou un lapin.

9. Animal selon l'une des revendications 1 à 8 **caractérisé en ce qu'**il produit une protéine comprenant la séquence SEQ ID N°2.

10. Animal selon l'une d es revendications 1 à 9 **caractérisé en ce qu'**il présente dans son génome la séquence d'acides nucléiques SEQID N°1.

11. Animal selon l'une des revendications 2 à 10 **caractérisé en ce que** l'expression du gène codant pour l'APP est sous le contrôle d'un promoteur exogène.

12. Animal selon l'une des revendications 1 à 11 **caractérisé en ce qu'**il présente une perte neuronale.

13. Animal selon l'une des revendications 1 à 10 **caractérisé en ce qu'**il présente un rapport Abeta42/Abeta total supérieur à environ 0,9.

14. Animal selon l'une des revendications 1 à 13 **caractérisé en ce que** la protéine préséniline 1 mutée est endogène.

15. Cellule ou lignée cellulaire dérivée d'un animal selon l'une des revendications 1 à 14.

16. Cellule ou lignée cellulaire comprenant une séquence d'acides nucléiques codant pour le gène murin de la préséniline 1 sous une forme mutée en M233T et L235P.

17. Cellule souche embryonnaire dérivée d'un animal selon l'une des revendications 1 à 14.

18. Protéine PS1 de souris portant les mutations des acides aminés M en T, et L en P respectivement aux positions 233 et 235.

19. Protéine selon la revendication 18 **caractérisée en ce qu'**elle comprend la séquence SEQ ID N°2.

20. Acide nucléique codant la protéine PS1 de souris portant les mutations des acides aminés M en T, et L en P respectivement aux positions 233 et 235.

21. Acide nucléique selon la revendication 20 **caractérisé en ce qu'**il comprend la séquence SEQ ID N°1.

22. Vecteur comprenant un acide nucléique selon la revendication 21.

23. Utilisation d'un animal selon l'une des revendications 1 à 14, pour la mise en évidence de composés destinés au traitement de la maladie d'Alzheimer.

24. Procédé de mise en évidence de composés destinés au traitement des maladies neurodégénératives comprenant les étapes suivantes :
- administration du composé ou d'un mélange de composés à tester à un animal selon l'une des revendications 1 à 14, et
- observation de l'évolution d'un ou plusieurs marqueurs caractéristiques reproduisant la neuropathologie observée chez l'homme.

25. Procédé de mise en évidence de composés destinés au traitement des maladies neurodégénératives comprenant les étapes suivantes :
- la mise en contact de cellules extraites d'un animal selon l'une des revendications 1 à 14 avec un composé ou un mélange de composés et
- la mesure du ou des effet(s) des composés au niveau des cellules entières, dans des homogénats de cellules ou sur une fraction subcellulaire.

26. Utilisation d'une protéine selon l'une des revendications 18 et 19 pour la mise en évidence de composés destinés au traitement de la maladie d'Alzheimer.

## Claims

1. Nonhuman animal exhibiting a nucleic acid sequence encoding a presenilin 1 protein carrying in the homozygous state, the mutations corresponding to the mutations M233T and L235P on the mouse PS1 protein.

2. Animal according to Claim 1, also comprising a nucleic acid sequence encoding all or part of the gene encoding APP.

3. Animal according to either of Claims 1 and 2, **characterized in that** it expresses an amount of mutated PS1 comparable to the amount of endogenous PS1 of an animal expressing a nonmutated PS1.

4. Animal according to one of Claims 1 to 3, **characterized in that** the presenilin 1 protein carrying the mutations M233T and L235P is of murine origin.

5. Animal according to one of Claims 2 to 4, **characterized in that** the APP protein is APP751 and is of human origin.

6. Animal according to one of Claims 2 to 5, **characterized in that** the APP751 protein is of human origin and exhibits the Swedish and London mutations.

7. Animal according to one of Claims 1 to 6, **characterized in that** it is a rodent.

8. Animal according to one of Claims 1 to 7, **characterized in that** it is a mouse, a rat or a rabbit.

9. Animal according to one of Claims 1 to 8, **characterized in that** it produces a protein comprising the sequence SEQ ID N° 2.

10. Animal according to one of Claims 1 to 9, **characterized in that** it exhibits in its genome the nucleic acid sequence SEQ ID N° 1.

11. Animal according to one of Claims 2 to 10, **characterized in that** the expression of the gene encoding APP is under the control of an exogenous promoter.

12. Animal according to one of Claims 1 to 11, **characterized in that** it exhibits a neuronal loss.

13. Animal according to one of Claims 1 to 10, **characterized in that** it exhibits an Abeta42/total Abeta ratio of greater than approximately 0.9.

14. Animal according to one of Claims 1 to 13, **characterized in that** the mutated presenilin 1 protein is endogenous.

15. Cell or cell line derived from an animal according to one of Claims 1 to 14.

16. Cell or cell line comprising a nucleic acid sequence encoding the murine presenilin 1 gene in an M233T- and L235P-mutated form.

17. Embryonic stem cell derived from an animal according to one of Claims 1 to 14.

18. Mouse PS1 protein carrying the amino acid mutations M to T, and L to P, respectively at positions 233 and 235.

19. Protein according to Claim 18, **characterized in that** it comprises the sequence SEQ ID N° 2.

20. Nucleic acid encoding the mouse PS1 protein carrying the amino acid mutations M to T, and L to P, respectively at positions 233 and 235.

21. Nucleic acid according to Claim 20, **characterized in that** it comprises the sequence SEQ ID N° 1.

22. Vector comprising a nucleic acid according to Claim 21.

23. Use of an animal according to one of Claims 1 to 14, for demonstrating compounds intended to treat Alzheimer's disease.

24. Method for demonstrating compounds intended for the treatment of neurodegenerative diseases, comprising the following steps:
- administering the test compound or a mixture of test compounds to an animal according to one of Claims 1 to 14, and
- observing the evolution of one or more characteristic markers reproducing the neuropathology observed in humans.

25. Method for demonstrating compounds intended for the treatment of neurodegenerative diseases, comprising the following steps:
- bringing cells extracted from an animal according to one of Claims 1 to 14 into contact with a compound or a mixture of compounds, and
- measuring the effect(s) of the compounds on whole cells, in cell homogenates or on a subcellular fraction.

26. Use of a protein according to either of Claims 18 and 19, for demonstrating compounds intended for the treatment of Alzheimer's disease.

## Patentansprüche

1. Nichtmenschliches Tier mit einer Nukleinsäuresequenz, die für ein Presenilin-1-Protein kodiert, das im homozygoten Zustand die Mutationen, die den Mutationen M233T und L235P auf dem PS1-Protein der Maus entsprechen, trägt.

2. Tier nach Anspruch 1, das weiterhin eine Nukleinsäuresequenz, die für die Gesamtheit oder einen Teil des Gens, das für APP kodiert, kodiert, umfaßt.

3. Tier nach einem der Ansprüche 1 und 2, **dadurch gekennzeichnet, daß** es eine Menge an mutiertem PS1 umfaßt, die mit der Menge an endogenem PS1 eines Tiers, das ein nichtmutiertes PS1 exprimiert, vergleichbar ist.

4. Tier nach einem der Ansprüche 1 bis 3, **dadurch gekennzeichnet, daß** das Presenilin-1-Protein, das die Mutationen M233T und L235P trägt, aus der Maus stammt.

5. Tier nach einem der Ansprüche 2 bis 4, **dadurch gekennzeichnet, daß** es sich bei dem APP-Protein um APP751 handelt und daß es aus dem Menschen stammt.

6. Tier nach einem der Ansprüche 2 bis 5, **dadurch gekennzeichnet, daß** das APP751-Protein aus dem Menschen stammt und die Mutationen "Swedish" und "London" aufweist.

7. Tier nach einem der Ansprüche 1 bis 6, **dadurch gekennzeichnet, daß** es sich um ein Nagetier handelt.

8. Tier nach einem der Ansprüche 1 bis 7, **dadurch gekennzeichnet, daß** es sich um eine Maus, eine Ratte oder ein Kaninchen handelt.

9. Tier nach einem der Ansprüche 1 bis 8, **dadurch gekennzeichnet, daß** es ein Protein, umfassend die Sequenz SEQ ID NO. 2, produziert.

10. Tier nach einem der Ansprüche 1 bis 9, **dadurch gekennzeichnet, daß** es in seinem Genom die Nukleinsäuresequenz SEQ ID NO. 1 aufweist.

11. Tier nach einem der Ansprüche 2 bis 10, **dadurch gekennzeichnet, daß** die Expression des Gens, das für APP kodiert, unter der Kontrolle eines exogenen Promoters steht.

12. Tier nach einem der Ansprüche 1 bis 11, **dadurch gekennzeichnet, daß** es Neuronenverlust aufweist.

13. Tier nach einem der Ansprüche 1 bis 10, **dadurch gekennzeichnet, daß** es ein Abeta42/Gesamt-Abeta-Verhältnis von über ungefähr 0,9 aufweist.

14. Tier nach einem der Ansprüche 1 bis 13, **dadurch gekennzeichnet, daß** das mutierte Presenilin-1-Protein endogen ist.

15. Zelle oder Zellinie, die von einem Tier nach einem der Ansprüche 1 bis 14 abstammt.

16. Zelle oder Zellinie, die eine Nukleinsäuresequenz umfaßt, welche für das Maus-Presenilin-1-Gen in einer Form mit den Mutationen M233T und L235P kodiert.

17. Embryonale Stammzelle, die von einem Tier nach einem der Ansprüche 1 bis 14 abstammt.

18. Maus-PS1-Protein, das in den Positionen 233 und 235 die Aminosäuremutationen M zu T bzw. L zu P trägt.

19. Protein nach Anspruch 18, **dadurch gekennzeichnet, daß** es die Sequenz SEQ ID NO. 2 umfaßt.

20. Nukleinsäure, die für das Maus-PS1-Protein, das in den Positionen 233 und 235 die Aminosäuremutationen M zu T bzw. L zu P trägt codiert.

21. Nukleinsäure nach Anspruch 20, **dadurch gekennzeichnet, daß** sie die Sequenz SEQ ID NO. 1 umfaßt.

22. Vektor, umfassend eine Nukleinsäure nach Anspruch 21.

23. Verwendung eines Tiers nach einem der Ansprüche 1 bis 14 zum Nachweisen von Verbindungen, die für die Behandlung von Morbus Alzheimer bestimmt sind.

24. Verfahren zum Nachweisen von Verbindungen, die für die Behandlung von neurodegenerativen Erkrankungen bestimmt sind, wobei das Verfahren die folgenden Schritte umfaßt:
- Verabreichen der Testverbindung oder einer Mischung von Testverbindungen an ein Tier nach einem der Ansprüche 1 bis 14, und
- Beobachten der Entwicklung von einem oder mehreren charakteristischen Markern, die die beim Menschen beobachtete Neuropathologie wiedergeben.

25. Verfahren zum Nachweisen von Verbindungen, die für die Behandlung von neurodegenerativen Erkrankungen bestimmt sind, wobei das Verfahren die folgenden Schritte umfaßt:
- Inkontaktbringen von Zellen, die einem Tier nach einem der Ansprüche 1 bis 14 entnommen wurden, mit einer Verbindung oder einer Mischung von Verbindungen, und
- Bestimmen der Wirkung(en) der Verbindungen auf die ganzen Zellen, in Zellhomogenaten oder auf eine subzelluläre Fraktion.

26. Verwendung eines Proteins nach einem der Ansprüche 18 und 19 zum Nachweisen von Verbindungen, die für die Behandlung von Morbus Alzheimer bestimmt sind.
